# EUROPEAN PATENT SPECIFICATION

(11) **EP 0 833 945 B1**
(45) Date of publication and mention of the grant of the patent: **30.01.2002**
(21) Application number: 96920611.9
(22) Date of filing: 03.06.1996
(51) Int. Cl.: C12Q 1/68, C12P 19/34

(54) **DNA POLYMERASE EXTENSION ASSAY**
DNA-POLYMERASE VERLÄNGUNGS ASSAY
PROCEDE DE DETECTION PAR EXTENSION CATALYSEE PAR L'ADN POLYMERASE

(30) Priority: 07.06.1995 US 487760
(43) Date of publication of application: 08.04.1998
(73) Proprietor: MERCK & CO., INC., Rahway, New Jersey 07065 (US)
(72) Inventor: COLE, James, L., Rahway, NJ 07065 (US); KUO, Lawrence, C., Rahway, NJ 07065 (US); OLSEN, David, B., Rahway, NJ 07065 (US)
(74) Representative: Hiscock, Ian James
(86) International application number: US9608330
(87) International publication number: WO9640994

(56) References cited:
- EP-A- 0 300 796
- WO-A-96/40993
- US-A- 4 521 509
- CHUNG T D ET AL: "Biochemical studies on capped RNA primers identify a class of oligonucleotide inhibitors of the influenza virus RNA polymerase." PROCEEDINGS OF THE NATIONAL ACADEMY OF SCIENCES OF THE UNITED STATES OF AMERICA, (1994 MAR 15) 91 (6) 2372-6, XP000600722
- KUPPUSWAMY ET AL.: "Single nucleotide primer extension to detect genetic diseases: Experimental application to hemophilia B (factor IX) and cystic fibrosis genes" PROCEEDINGS OF THE NATIONAL ACADEMY OF SCIENCE, USA, vol. 88, February 1991, pages 1143-1147, XP000371662
- CELL, March 1981, Vol. 23, PLOTCH et al., "A Unique Cap(m7GpppXm)-Dependent Influenza Viron Endonuclease Cleaves Capped RNAs to Generate the Primers that Initiate Viral RNA Transcription", pages 847-858.
- ANTIMICROB. AGENTS CHEMOTHER., December 1994, Vol. 38, Number 12, TOMASSINI et al., "Inhibition of Cap(m7GpppXm)-Dependent Endonuclease of Influenza Virus by 4-Substituted 2,4-Dioxobutanoic Acid Compounds", pages 2827-2837.
- VIROLOGY, April 1995, Vol. 208, SHI et al., "Influenza A Virus RNA Polymerase Subunit PB2 is the Endonuclease which Cleaves Host Cell mRNA and Functions Only as the Trimeric Enzyme", pages 38-47.
- ANALYT. BIOCHEM., November 1995, Vol. 231, COLE et al., "Assay for Influenza Virus Endonuclease Using DNA Polymerase Extension of a Specific Cleavage Product", pages 309-314.

## Description

### FIELD OF THE INVENTION

This invention relates to a novel sensitive assay to detect and quantify the presence of an enzyme which acts to produce a single-stranded oligonucleotide product, or to inhibitors of such enzyme.

### BACKGROUND OF THE INVENTION

Enzymes which use polynucleotides as substrates are often fundamental to the biochemical function of many organisms and viruses. This group of enzymes includes, for example, endonucleases, exonucleases and ribozymes. A number of enzymes act on substrates to give single-stranded oligonucleotide products. For example, the influenza virus endonuclease cleaves capped host cell transcripts 10 to 13 bases from the 5' end. Sequence specific RNA endonucleases are known; for example the 2-5A-dependent RNase found in higher animals functions to cleave single-stranded regions of RNA 3' of UpNp dimers, with a preference for UU and UA sequences. In addition, ribozymes are known to cleave single-stranded RNAs at specific recognition sequences.

While it is recognized that inhibitors or activators of these enzymes might be new classes of therapeutic or preventative compounds, particularly against viral diseases, identification of such compounds has been hampered by the lack of a convenient assay system.

An ideal enzyme assay system should have: a) high throughput; b) the ability to distinguish enzyme-catalyzed cleavage from nonspecific nucleotide cleavage; and c) high sensitivity. Previous nucleotide cleavage assays involved the use of polyacrylamide gel electrophoresis to separate product from substrate (Plotch *et al.*, 1981 *Cell 23*: 847-858) which is not convenient for processing large numbers of samples.

### DETAILED DESCRIPTION OF THE INVENTION

This invention is directed to a novel, accurate, sensitive, rapid assay which is specific for a chosen enzyme which acts on a substrate to produce a single-stranded oligonucleotide product. This invention comprises a method of measuring the enzyme activity of a sample suspected of containing either an enzyme which acts on an oligonucleotide substrate to generate a single-stranded oligonucleotide product, or an inhibitor of such enzyme comprising:
a) adding the oligonucleotide substrate to the sample whose activity is to be assayed to generate the single-stranded oligonucleotide product;
b) hybridizing the single-stranded oligonucleotide product with a DNA template, said DNA template comprising a first segment substantially complementary to the oligonucleotide product and a template 5'-extension region attached to the first segment, said extension region comprising at least one nucleotide, under hybridization conditions to form a RNA: DNA heteroduplex or a DNA: DNA duplex;
c) adding labeled mononucleotide which is complementary to the second segment of the DNA template;
d) adding a DNA polymerase to the heteroduplex or duplex under conditions permitting the DNA polymerase to catalyze the addition of the labeled mononucleotide to the 3'-end of the oligonucleotide product to produce a labeled polymerase product; and
e) measuring the amount of labeled polymerase product as a measure of the amount of enzyme activity of the sample.

A further embodiment of this invention is a method of determining the site where an endonuclease cleaves an oligonucleotide substrate to create a single-stranded cleavage product comprising
a) providing at least one DNA template to a sample, wherein the DNA template comprises a first 3'-segment and a 5'-extension region, provided that the 3'-segment is thought to be complementary and of the same length as the cleavage product, and said 5' extension region comprises at least one nucleotide;
b) adding cleavage product to the sample under hybridizing conditions to form a RNA: DNA heteroduplex or DNA: DNA duplex structure;
c) adding to the sample a labeled mononucleotide which is complementary to the 5'-extension region;
d) adding to the sample a DNA polymerase under conditions permitting a polymerase reaction to occur wherein the labeled mononucleotide is added to the 3'-end of the cleavage product if the 5'-segment of the DNA template is complementary and of the same length as the cleavage product; and
e) determining if the reaction of step d) occurs.

In accordance with this invention, the sample to be analyzed may contain an unknown quantity of an enzyme which acts on an oligonucleotide substrate (either DNA or RNA) to produce a single-stranded oligonucleotide product. Examples of such enzymes include various endonucleases, including either DNA or RNA endonucleases and ribozymes. Especially preferred are viral endonucleases and ribozymes.

In one embodiment of the invention, the amount of enzyme present in a sample may be quantified. In an alternative embodiment, the sample may contain a known amount of enzyme, and a substance whose inhibitory activity is to be determined. The amount of labeled polymerase product is compared to the amount of labeled polymerase product produced by a control sample where no inhibitors were present, and the degree of inhibitory activity present in the sample can be determined.

In another aspect of this invention, the assay can be used to easily identify the position where the enzyme cleaves its substrate. In this embodiment of the assay, at least one, and preferably a series of DNA templates is made, each having a different length, and each having a 5'-extension region. Endonuclease cleavage product is added. If the DNA template extension region is complementary to the cleavage product and the same length as the cleavage product, it will hybridize and a polymerase addition reaction can occur. If the template extension region is not the same length as the cleavage product so that the 3'-OH of the cleavage product cannot prime polymerization, the polymerase reaction will not occur.

In another embodiment of this invention, ribozyme activity, rather than endonuclease activity is determined using this assay.

To determine the amount of labeled polymerase product present, any detection method may be employed. A preferred method is to pass the sample containing labeled polymerase product and excess labeled mononucleotide through a filter which traps the labeled polymerase product and determining the amount of labeled polymerase product which has been trapped on the filter.

### BRIEF DESCRIPTION OF THE FIGURES

FIGURE 1 is a flowchart illustrating the DNA polymerase extension assay of this invention.

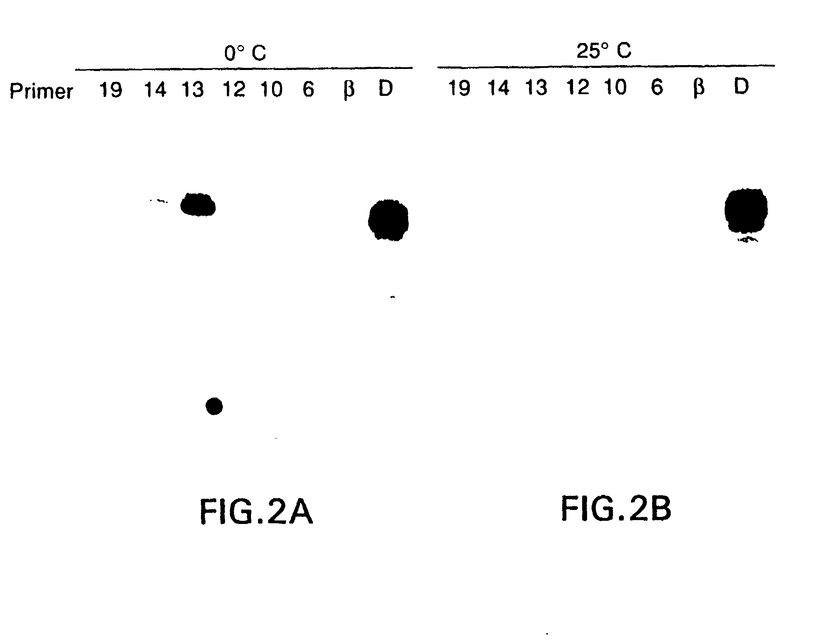

FIGURE 2 is a photograph of a phosphorimager scan of a 20% acrylamide 8M urea polyacrylamide gel demonstrating the effects of primer length and temperature on polymerase-catalyzed extension reactions. The lane labeled 19 corresponds to an uncapped RNA primer (19 nt) with the sequence of the AlMV substrate (5'-GUUUUUAUUUUUAAUUUUC-3') (SEQ.ID.NO.:1); lanes labeled 14-6 correspond to RNA primers of the indicated length derived from 3' deletions of the 19 nt primer. The lane labeled β corresponds to a 13 nt RNA derived from the 5' end of β-globin mRNA (5'-ACACUUGCUUUUG-3') (SEQ.ID.NO.:2). The lane labeled D corresponds to a 13 nt DNA with the AlMV primer sequence (5'-GTTTTTATTTTTA-3') (SEQ.ID.NO.:3).

FIGURE 3 is a graph demonstrating the linear response of DNA polymerase amplification. Reactions were carried out as described in Example 2. The line drawn through the points is a linear least-squares fit to the data with a correlation coefficient of 0.9999

FIGURE 4 is a gel demonstrating polymerase-catalyzed extension of the influenza endonuclease cleavage product, as described in Example 1.

### DEFINITIONS

Throughout the specification and claims the following definitions shall apply:
"nt" is the abbreviation for nucleotide.
"AlMV" is the abbreviation for Alfalfa Mosaic Virus.
"Substantially complementary" means that there is sufficient complementarity so that under the experimental conditions employed, the 3' region of the primer will hybridize with the DNA template strand such that the duplex so formed is capable of serving as a substrate for a DNA polymerase. "Substantially complementary" further requires that there is sufficient complementarity so that a duplex can form and that the duplex has sufficient stability so that its melting temperature is greater than the freezing point of the solvent system. In cases where the substrate is RNA, then "substantially complementary" means that there is sufficient complementarity so that the 3'-region of the RNA primer will hybridize with the DNA template strand such that the heteroduplex is capable of serving as a substrate for a DNA polymerase; further there is sufficient complementarity so that the heteroduplex will form and it has sufficient stability so that its melting temperature is greater than the freezing point of the solvent system.
"Oligonucleotide" is at least two nucleotides in length.
"Substantially repeated" means that the oligonucleotide is made up of at least 80% of the same base, preferably at least 90% of the same base, and even more preferably at least 95% of the same base.
"Derived from AlMV" means that the oligonucleotide is at least 80% homologous to the 5'-end of the AlMV 4 RNA, and is able to act as a substrate of influenza endonuclease, i.e. it can be cleaved by influenza endonuclease.
"Ribozyme" is a type of enzyme which is made of RNA rather than protein.
"Enzyme" is meant to include various biological molecules having catalytic functions, regardless of whether the molecule is proteinaceous, and specifically includes ribozymes.

The substrate can be any oligonucleotide substrate, either DNA or RNA, which is acted upon by an enzyme to produce a single-stranded oligonucleotide product. It may be modified, i.e., by a 5'-cap, if the enzyme requires this type of substrate. In one embodiment, the assay uses 5'-capped RNA which is cleaved by the influenza endonuclease. A 5'-capped RNA precursor may be utilised to make a 19 nt RNA containing a 5'-triphosphate. This is then treated with a guanylyltransferase. Guanylyl transferases are well known and may be obtained commercially; for example, Vaccinia virus guanylyltransferase may be purchased from Bethesda Research Laboratories, Gaithersburg, MD. The result is a 19 nt 5'- capped RNA substrate which is cleaved by the influenza endonuclease, generating a 13 nt product. Preferably, the endonuclease substrate is derived from the 5' end of the AlMV 4 RNA, which has been previously demonstrated to be a substrate for the influenza endonuclease and is cleaved at nucleotide A13.

In an alternate preferred embodiment, the enzyme is a ribozyme, such as a hammerhead ribozyme, hairpin ribozyme or a Group I intron type ribozyme and the substrate is an RNA molecule. As ribozymes generally require the presence of a divalent ion to function, it is important that these be present in the assay reaction mixture. Depending on the particular ribozyme chosen, the known important ions which are needed for activity include: divalent metal cations such as Zn⁺², Mn⁺², Mg⁺², or Ca⁺². The oligoribonucleotide substrate is then cleaved by the ribozyme in the presence of the cation.

The oligonucleotide product is then used as a primer for a DNA polymerase-catalyzed extension reaction. In order for the polymerase extension reaction to proceed, a DNA template is required. The DNA template is a two-part molecule it contains a 3'-region which is substantially complementary to the oligonucleotide product, joined to a template 5'-extension region. Although in theory the 5'-extension region may be made from any desired nucleotides, it is preferred that they be substantially repeated nucleotides, and that the nucleotides present in the extension region are not present in the 3'-complementarity region in order to achieve specificity for product cleaved at the correct position. In the most preferred embodiment, the 5'-extension region is made up of repeated nucleotides. If the 5'-extension region is not made from substantially repeated nucleotides, then there may be specificity problems with the assay, unless the extension region is quite long to amplify the signal of the correct cleavage product relative to nonspecific products.

There is no particular limit as to the length of the extension region; it may be as short as a single nucleotide or as long as existing synthesis methods permit (up to or exceeding 50 nucleotides). In preferred embodiments, the 5'- extension region is at least one nucleotide residue in length, and is more preferably at least about 10 residues in length. Thus, the template extension region should preferably be a poly-dC, a poly-dG, a poly-dA, or a poly-dT region. In one preferred embodiment of this invention, the template extension region is a poly-dC residue 10 nt in length.

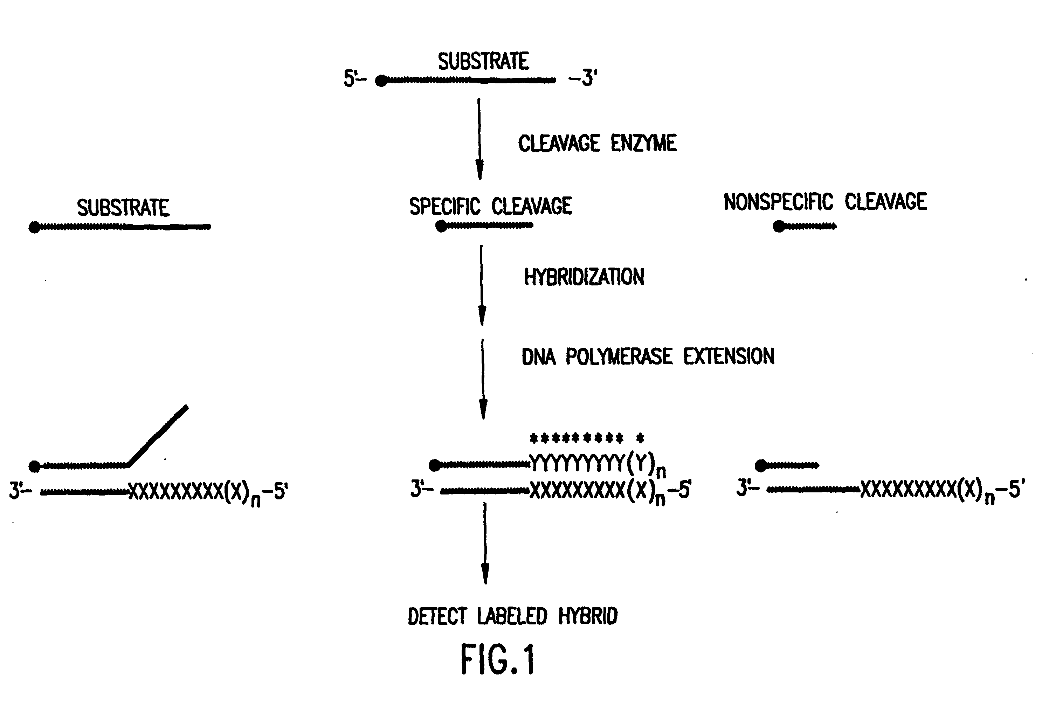

During the DNA polymerase-catalyzed extension reaction, the oligonucleotide product will be extended in length by labeled nucleotides which are complementary to the nucleotides present in the DNA template extension region. For example, if the template extension region is a 10 residue poly-C, the oligonucleotide product will be extended 10 bases by a labeled poly-G sequence. While any type of label which is conventionally used in the nucleotide assay arts may be used to label the nucleotides which will be incorporated into the product extension region, such as fluorescent or absorption labels, it is preferred to use a radiolabel. In a preferred embodiment, the product extension region is poly-α-³²P labeled dGMP (as shown in Figure 1).

In addition, to prevent a polymerase-catalyzed extension from occurring on the 3'-OH end of the template, the 3'-OH of the primer is preferably blocked. Numerous blocking materials are known and suitable, and include cordycepin (3'-deoxyadenosine) and other 3'-moieties without a 3'-OH. In one embodiment of this invention, the 3'-OH is blocked with a (3-amino-2-hydroxy)-propoxyphosphoryl. In another embodiment of this invention, the 3'-OH is blocked by introduction of a 3'-3'-A-5' linkage. While it is possible to run an assay of this invention without blocking the 3'-OH of the primer, blocking the 3'-OH end helps to prevent background signals and this helps to increase the sensitivity of the assay.

Numerous DNA polymerase enzymes are known and may be used in the DNA polymerase reaction step of this invention. In one embodiment, where the enzyme substrate is an RNA, Sequenase® Version 2, a mutant of bacteriophage T7 DNA polymerase (obtained from United States Biochemicals, Cleveland, Ohio) in which the 3' to 5' exonuclease activity is abolished by *in vitro* mutagenesis (Tabor *et al.*, 1989 *J. Biol. Chem 264*:6447-6458) is a preferred DNA polymerase. This polymerase is preferred because it is able to use oligoribonucleotides as primers, it replicates with high fidelity and does not have a 3' to 5' "proofreading" activity.

Depending on the type of label used, detection of the labeled hybrid polymerase product may be achieved by any appropriate means. Generally this may include a step of separating labeled mononucleotide from labeled hybrid polymerase product. In a preferred embodiment, detection is achieved by filtering the sample mixture (which at this point in the assay method, contains the labeled hybrid polymerase product and excess labeled mono-nucleotide) through a nylon membrane. The unincorporated labeled mononucleotides flow through the membrane while the labeled hybrid polymerase reaction product is captured. If the label was a radiolabel, then the amount of radioactivity bound to the filter may be quantified using a phosphorimager or by a plate reading scintillation counter.

This assay has distinct advantages over the prior art assays in that it does not involve an electrophoresis step and may be run in 96-well microtiter plate format. Other key advantages of the assay of this invention are that it monitors the substrate cleavage reaction only at the correct position in the sequence, thereby discriminating against nonspecific cleavage products. Also importantly, this assay is sensitive enough to detect 200 attomoles (2 x 10⁻¹⁶ moles) of product generated in a typical cleavage reaction.

The specificity of the preferred embodiment assays of this invention is achieved through the choice of the DNA template and the high fidelity of the T7 DNA Sequenase® polymerase. Under the conditions of a typical reaction more than 90% of the substrate is not cleaved, but this does not interfere with the specificity of the assay because the uncleaved RNA substrate does not serve as a primer for polymerization since the 3' end cannot base-pair to the template. In addition, nonspecific cleavage products, such as those which have been cleaved at a site different than the enzyme cleavage site are not extended. Although these shorter (or longer) sequences may hybridize with the template, they are not extended by the DNA polymerase because this reaction would require the presence of nucleotides which are not present in the reaction mixture.

The polymerase extension reaction is dependent on hybridization of the primer onto the DNA template. Therefore, for effective hybridization of the primer to the DNA template, polymerase extension reactions are preferably carried out at least 10°C below the melting temperature of the primer:template complex. A typical preferred reaction temperature is 0°C for a primer:template complex with a melting temperature of 14°C.

Figure 2 illustrates the specificity of the polymerase-catalyzed extension reaction and the effects of temperature. In an influenza endonuclease assay, a capped AlMV RNA substrate which is cleaved into a 13 nt RNA product is used. Synthetic, uncapped RNA and DNA primers of various lengths and sequence were hybridized with the DNA template and extended with polymerase and α³²P-dGTP. When the reaction is carried out at 0°C the only primers that give efficient extension are the 13 nt AlMV RNA and the corresponding sequence in DNA. Although incompletely extended products are visible on the gel, the reaction conditions have been optimized such that the most intense band corresponds to complete primer extension. A fainter band above the most intense band corresponds to a single base addition. Several DNA polymerases are known to catalyze the addition of a single base onto the 3'-OH termini of a blunt-ended DNA (Clark, 1988 *Nucl. Acids Res. 16*:9677-9686); presumably, a similar activity is also present in the Sequenase® polymerase that was used. The much less intense bands in the lane containing 14 nt RNA primer are attributable to a small contaminant of the 13 nt RNA present in the 14 nt ribonucleotide. No extension products are observed using either the full length 19 nt primer, AIMV primers smaller than 13 nt, or a heterologous 13 nt RNA derived from the 5' end of the β-globin transcript (5'-ACACUUGCUUUUG-3') (SEQ.ID. NO.:2). The Polymerase® reaction selectively extends the AlMV 13 nt primers and discriminates against sequences corresponding to uncleaved AIMV substrate or shorter nonspecific cleavage products. It is noteworthy that the high fidelity of Sequenase® prevents incorrect extension of AlMV primers differing in length by as little as a single base.

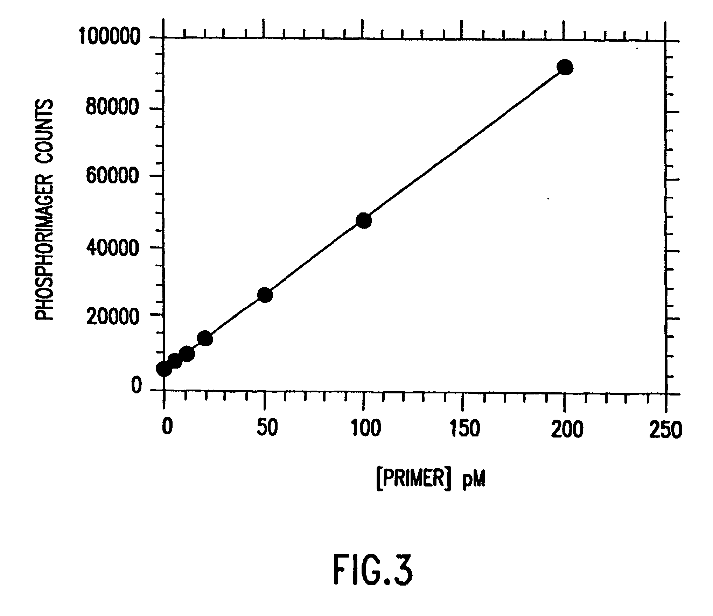

It has been found in accordance with this invention that the polymerase extension reaction can be conveniently monitored by filtration through nylon membranes. Under the conditions described, quantitative binding of the extended product can be observed, with only a small amount (less than 0.0003%) of the unincorporated α³²P-dGTP being retained on the nylon membrane. For high volume screening purposes, the filtration process can be performed using a 96-well manifold and the filter bound radioactivity can be quantitated with either a phosphorimager device or a microplate scintillation counter. Figure 3 shows the sensitivity and linear response using nylon membrane filtration and phosphorimager detection for extension of the 13 nt AlMV RNA primer. Excellent linearity is observed up to 200 pM primer in a reaction volume of 20 µL. Note that under typical assay conditions approximately 10% of the substrate will be converted to hybrid polymerase extension reaction product, corresponding to 40 pM of product; this amount is well within the linearity range and detection limits of the assay.

A background primer-independent reaction is observed which is manifest as a faint band running above the major extension products across all lanes in Figure 2 and as a nonzero y-intercept in Figure 3. In the absence of template, the former background signal is absent and the latter is dramatically reduced, suggesting that they are correlated. The background signals are independent of the presence of influenza core protein but they are dependent on Sequenase®. Thus, the background is due to a Sequenase®-catalyzed addition of α-³²P dGTP to the single-stranded template. Much higher background signals are observed using a template in which the 3'-OH group is not blocked by an aminolinker moiety, suggesting that the reaction requires a free 3'-OH. The background signals can be further reduced by treatment of the aminolinker-blocked template with ddATP and terminal deoxy-nucleotidyl transferase, which suggests that presence of a free 3'-OH containing impurity in the template.

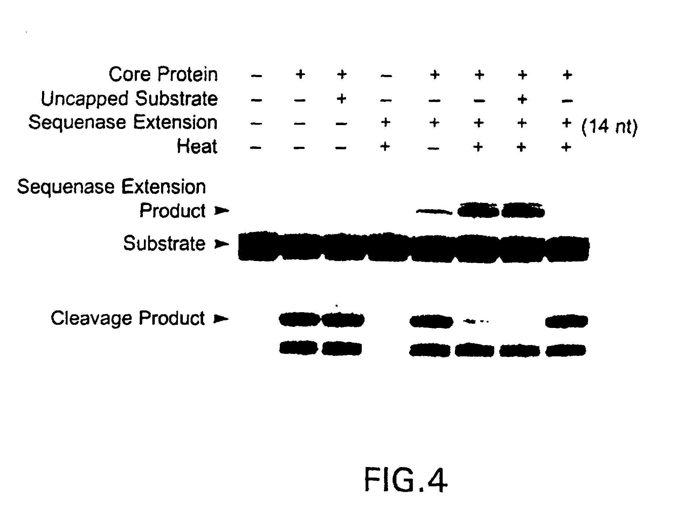

In order to clearly characterize the coupled influenza endonuclease/polymerase extension process, a series of reactions were carried out using m⁷G³²p- labeled AlMV substrate and unlabeled dGTP and were analyzed by gel electrophoresis. Figure 4 shows that in the absence of core protein the AIMV substrate migrates as a single band corresponding to a length of 19 nt. Incubation of substrate with influenza core protein results in a major cleavage product as well as minor products that are shorter. The major product corresponds to cleavage at A13; this product is extended upon incubation with GTP. Similar results have previously been reported using the full-length AlMV RNA 4 (Plotch *et al.*, 1989 *Cell 23*:847-858). The addition of a 10-fold excess of unlabeled, uncapped AlMV substrate does not affect the extent of cleavage. Incubation of substrate with Sequenase® , template and dGTP does not result in any extension in the absence of influenza core protein. Cleavage of substrate by core protein following by polymerase extension results in the appearance of a faint band above the substrate band. This band corresponds to the addition of 10 dG residues to the 13 nt product. Note that in this sample the bulk of the cleavage product is not extended by Sequenase® . In contrast, in the sample which is incubated at 80°C for 1 minute following cleavage, near-quantitative extension is observed. The dissociation of the cleavage product is likely to be quite slow and the heating step may serve to release the bound product by denaturing the influenza endonuclease complex. A faint band observed above the polymerase extension product likely corresponds to addition of an extra dG residue to the blunt-ended extended primer:template duplex, as was observed in Figure 2. As in the case of the cleavage reaction alone, the polymerase extension assay is not affected by the presence of a 10-fold excess of unlabeled, uncapped AlMV substrate. In the last lane, the 24 nt template that contains a 14 nt complementary region was added instead of the 13 nt complementary template. The specific cleavage product is not extended in this sample, which confirms that the major product corresponds to cleavage at A13 and not at A14.

The DNA polymerase extension assay was validated for detecting influenza endonuclease inhibitors by using the inhibitor, (4-[N-benzene-sulfonyl-3-(4-chlorobenzyl)piperidin-3-yl]-2,4-dioxo-butanoic acid), which we have previously identified using the gel-based assay. This compound is similar to the 4-substituted 2,4-dioxobutanoic acids recently described as inhibitors of the influenza endonuclease (Tomassini *et al.*, 1994 *AntiMicrob*. *Agents Chemother. 38*:2827-2837). Titrations of the inhibition of the endonuclease by this compound were performed using a gel based assay and the DNA polymerase extension assay of this invention under the same experimental conditions. The IC₅₀ values determined by fitting the data obtained over a range of 3 nM to 10 µM to a simple hyperbolic inhibition model are 260 ± 60 nM for the gel assay and 220 ± 50 nM for the DNA polymerase extension assay. Within error, the potency of this compound is the same in both assays, indicating that the DNA polymerase extension assay accurately monitors inhibition of the influenza endonuclease.

In another aspect of this invention, the DNA polymerase extension assay provides a convenient method to determine the cleavage position of capped substrates. In the past, the site of cleavage by an endonuclease was determined by generating sequence ladders with alkaline digestion or by digestion with RNases and electrophoresis. The former method suffers from ambiguities arising from lability of the m7G residue in the cap structure to alkaline hydrolysis and the latter from the faster electrophoretic mobility of the 3'-phosphorylated sequences generated by-RNases relative to the unphosphorylated 3'-OH ends generated by the endonuclease. In contrast, the cleavage site can be unambiguously defined using the DNA polymerase extension assay of this invention by comparing the extension reactions catalyzed using templates with complementary regions corresponding to the expected cleavage products. Figure 4 illustrates this aspect of the invention, where the template with a 13 nt complementary region serves as a substrate, whereas the 14 nt complementary template does not.

Another major advantage of the extension assay of this invention over existing methodologies is that the oligonucleotide product of interest can be detected in the presence of other nucleotides provided that they do not serve to prime polymerization. Thus, precise measurements of the reaction of interest can be performed even in complex, impure preparations. This is particularly helpful in monitoring ribozyme-mediated cleavage reactions.

The following non-limiting Examples are presented to better illustrate the invention.

### EXAMPLES

General Experimental Methods: Oligo-deoxyribonucleotides and uncapped oligo-ribonucleorides were synthesized by Midland Ceitified Reagent Company (Midland, TX) and were purified by anion-exchange HPLC. Triphosphorylated oligonucleotides were synthesized and capped. The sequence of the 19 nt substrate oligo-ribonucleotide is 5'-ppp-G(2'-OMe) UUUUUA-UUUUUAAUUUUC-3' (SEQ.ID. NO.:1). 3'-Truncated ribonucleotides were also synthesized which correspond to the 5'-region of the substrate with lengths of 14, 13, 12, 10 and 6 nt. Unless otherwise indicated, a 23 nt template was used in all experiments with the sequence: 5'-Biotin-CCCCCCCCCCTAAAA-ATAAAAAC-amino-3' (SEQ.ID.NO.:4), where the 5'-biotin is N-biotinyl-6-aminohexyloxyphosphoryl moiety and the 3'-amino is 3-amino-2-hydroxy-propoxyphosphoryl. For some experiments a 24 nt template was used with the sequence: 5'-Biotin-CCCCCCCCCC-TTAAAAATAAAAAC-amino-3' (SEQ.ID.NO.:5). α-³²P-dGTP (3000 Ci/mmole) was obtained from Dupont NEN. Unlabeled dGTP was obtained from Pharmacia. Sequenase® Version 2.0 was obtained from United States Biochemicals (Cleveland, OH).

Unless otherwise noted, the polymerase extension reactions contained I nM primer, 50 nM template, 500 nM Sequenase® , and 500 nM dGTP and were carried out for 2 hours at the indicated temperature. The reactions were analyzed on 20% polyacrylamide 8M urea gels.

### EXAMPLE 1

### Cleavage Reactions and DNA Polymerase Reactions

Influenza cleavage reactions were performed at 25°C in a buffer containing 100 mM tris[hydroxymethyl]aminomethane (Tris), pH 8.0, 50 mM KCI, 0.25 mM MgCl₂, 5 mM dithiothreitol (DTT), 4% (v/v) dimethyl sulfoxide (DMSO), in DEPC treated water. Polymerase extension reactions were performed in the same buffer, except that the MgCl₂ concentration was increased to 10 mM and the DMSO concentration to 9%. 500 nM dGTP was employed in extension reactions using end-labeled primers and a mixture of 50 nM α-³²P-dGTP and 450 nM unlabeled dGTP was employed with unlabeled primers. Unless otherwise indicated, extension reactions were performed at 0°C. For the inhibition measurements, the compound 4-[N-benzenesulfonyl-3-(4-chlorobenzyl)piperidin-3-yl]-2,4-dioxo-butanoic acid was preincubated with influenza core protein for 10 min. The cleavage reactions were initiated by adding 0.4 nM of unlabeled (gel assay) or end-labeled (DNA polymerase extension assay) substrate in a 15 µL volume reaction containing 0.75 µL influenza core protein and were run for 10 min at 25°C. For the DNA polymerase extension assay, the extension reaction was carried out in a 20 µL volume containing 50 nM Sequenase® , 500 nM dGTP, 50 nM template for 18 h at 0°C.

Reactions were analyzed either by electrophoresis in 20% polyacrylamide gels containing 8 M urea or by filtering through 0.2 µm pore Nytran® membranes in a 96-well manifold (Schleicher and Schuell, Keene, NH). For filtration, samples were diluted with 200 µL of 250 mM EDTA, pH 8.0 and 200 µL was loaded onto the membrane equilibrated in 5X SSC (0.75 M NaCl, 75 mM sodium citrate, pH 7.0) and filtered immediately. Each well was washed five times with 200 µL of 5X SSC, and the filter was removed from the manifold washed 3 times in 100 ml of 5X SSC.

Gels were visualized and filters were quantitated using a Phosphorimager® (Molecular Dynamics, Sunnyvale, CA) with the Imagequant® software provided by the manufacturer. In some experiments, the Nytran® filters were also quantitated using a TopCount® (Packard Instruments, Meriden, CT) microplate scintillation counter using Flexifilter® kits and Microscint® O scintillation fluid.

### EXAMPLE 2

### DNA Polymerase Extension Assay For Ribozyme Cleavage.

Kinetic reactions are performed according to the procedure of Fedor and Uhlenbeck 1990, *Proc. Natl. Acad. Sci USA* 87:1668-1672, using ribozyme concentrations of 1 nM, 10 mM divalent metal ion and RNA substrate concentrations of 50 nM. The DNA polymerase extension reaction is carried out using 50 nM Sequenase® , 500 nM labeled dATP, 100 nM capture oligonucleotide/template for 18 hours at 0°C. Reactions are analyzed by the methods described above.

## Claims

1. A method of measuring enzyme activily of a sample, wherein said enzyme acts on an oligonucleotide substrate to generate a single-stranded oligonucleotide product comprising:
a) adding the oligonucleotide substrate to the sample to be assayed to generate the oligonucleotide product;
b) hybridizing the oligonucleotide product with a DNA template, said DNA template comprising a first segment substantially complementary to the oligonucleotide product and a template 5'-extension region attached to the first segment, said 5'-extension region comprising at least one nucleotide, under hybridization conditions to form a RNA: DNA heteroduplex or a DNA: DNA duplex structure;
c) adding labeled mononucleotide which is complementary to the 5'-extension region of the DNA template;
d) adding a DNA polymerase to the heteroduplex or duplex structure under conditions permitting the DNA polymerase to catalyze the addition of the labeled mononucleotide to the 3'-end of the oligonucleotide product to produce a labeled polymerase product; and
e) measuring the amount of labeled polymerase product as a measure of the amount of enzyme activity of the sample.

2. A method according to Claim 1 wherein the enzyme is an endonuclease.

3. A method according to Claim 2 wherein the substrate is a DNA substrate.

4. A method according to Claim 2 wherein the substrate is an RNA substrate.

5. A method according to Claim 1 wherein the enzyme is a ribozyme and the sample further comprises a divalent cation.

6. A method according to Claim 1 wherein the template extension region is at least about 10 nucleotides in length.

7. A method according to Claim 6 wherein the template extension region comprises substantially repeated nucleotides.

8. A method according to Claim 1 wherein the labeled mononucleotides are radiolabeled.

9. A method according to Claim 8 wherein the amount of labeled polymerase product is measured by passing the sample comprising labeled polymerase product and excess labeled mononucleotide through a filter, so that the labeled polymerase product is captured in the filter, and measuring the amount of radiolabel present on the filter.

10. A method according to Claim 9 wherein the filter is a nylon filter.

11. A method according to Claim 1 wherein the sample to be assayed contains a putative inhibitor of the enzyme.

12. A method according to Claim 11, further comprising comparing the amount of enzyme activity of the sample to the activity determined for a control sample comprising the same enzyme which is not in the presence of a putative inhibitor.

13. A method according to Claim 5 wherein the sample to be assayed contains a putative inhibitor of ribozyme activity.

14. A method according to Claim 13, further comprising comparing the amount of ribozyme activity of the sample to the activity determined for a control sample comprising the same ribozyme which is not in the presence of a putative inhibitor.

15. The method of claim 1, wherein said enzyme is an RNA endonuclease and said sample further comprises a putative inhibitor of the endonuclease,
said oligonucleotide substrate is an RNA molecule,
said 5'-extension region comprises at least 10 substantially repeated nucleotides,
said labeled nucleotide is radiolabeled and complementary to said at least 10 substantially repeated nucleotides,
said labeled polymerase product is a radiolabeled polymerase product comprising said radiolabel, and wherein said step (e) comprises
(i) filtering the sample containing the radiolabeled polymerase product and excess radiolabeled mononucleotide by passing the sample through a nylon filter so that the radiolabeled polymerase product is trapped by the filter; and
(ii) measuring the amount of radiolabeled polymerase product trapped on the filter and comparing that amount to that obtained when no endonuclease inhibitor is present in a control sample.

16. The method of claim 1, wherein said enzyme is a ribozyme and said sample further comprises a putative inhibitor of said ribozyme,
said oligonucleotide substrate is an RNA molecule,
said 5'-extension region comprises at least 10 substantially repeated nucleotides,
said labeled nucleotide is radiolabeled and complementary to said at least 10 substantially repeated nucleotides,
said labeled polymerase product is a radiolabeled polymerase product comprising said radiolabel,
said sample comprises divalent cations for ribozyme activity, and wherein said step (e) comprises
(i) filtering the sample containing the radiolabeled polymerase product and excess radiolabeled mononucleotide by passing the sample through a nylon filter so that the radiolabeled polymerase product is trapped by the filter; and
(ii) measuring the amount of radiolabeled polymerase product trapped on the filter and comparing that amount to that obtained when no ribozyme inhibitor is present in a control sample.

17. A method of determining the site where a nuclease cleaves an oligonucleotide substrate to create a single-stranded cleavage product comprising:
a) providing at least one DNA template to a sample, wherein the DNA template comprises a first 3'-segment and a 5'-extension region, provided that the 3'-segment is thought to be complementary and of the same length as the cleavage product, and said 5' extension region comprises at least one nucleotide;
b) adding cleavage product to the sample under hybridizing conditions to form a RNA: DNA heteroduplex or DNA: DNA duplex structure;
c) adding to the sample a labeled mononucleotide which is complementary to the 5'-extension region;
d) adding to the sample a DNA polymerase under conditions permitting a polymerase reaction to occur wherein the labeled mononucleotide is added to the 3'-end of the cleavage product if the 5'-segment of the DNA template is complementary and of the same length as the cleavage product; and
e) determining if the reaction of step d) occurs.

18. A method according to Claim 17 wherein a plurality of DNA templates are provided to the sample in step (a), where the plurality of DNA templates each comprise said 5'-extension region and a second 3'-segment, wherein said second 3'-segment differs in length from said first 3'-segment.

## Patentansprüche

1. Verfahren zur Messung der Enzymaktivität einer Probe, wobei das Enzym auf ein Oligonukleotid-Substrat einwirkt, um ein einzelsträngiges Oligonukleotid-Produkt zu erzeugen, umfassend:
a) Zugeben des Oligonukleotid-Substrats zu der zu untersuchenden Probe, um das Oligonukleotid-Produkt zu erzeugen;
b) Hybridisieren des Oligonukleotid-Produkts mit einer DNA-Matrize, welche DNA-Matrize einen ersten Abschnitt, der im wesentlichen komplementär zu dem Oligonukleotid-Produkt ist, und einen Matrizen-5'-Verlängerungsbereich, der mit dem ersten Abschnitt verknüpft ist, wobei der 5'-Verlängerungsbereich mindestens ein Nukleotid umfaßt, umfaßt, unter Hybridisierungsbedingungen zur Bildung einer RNA:DNA-Heteroduplex- oder einer DNA:DNA-Duplex-Struktur;
c) Zugeben eines markierten Mononukleotids, welches komplementär zu dem 5'-Verlängerungsbereich der DNA-Matrize ist;
d) Zugeben einer DNA-Polymerase zu der Heteroduplex- oder Duplex-Struktur unter Bedingungen, welche es der DNA-Polymerase ermöglichen, die Addition des markierten Mononukleotids zu dem 3'-Ende des Oligonukleotid-Produkts zu katalysieren, um ein markiertes Polymeraseprodukt zu erzeugen; und
e) Messen der Menge an markiertem Polymeraseprodukt als Maß der Menge an Enzymaktivität der Probe.

2. Verfahren nach Anspruch 1, worin das Enzym eine Endonuklease ist.

3. Verfahren nach Anspruch 2, worin das Substrat ein DNA-Substrat ist.

4. Verfahren nach Anspruch 2, worin das Substrat ein RNA-Substrat ist.

5. Verfahren nach Anspruch 1, worin das Enzym ein Ribozym ist und die Probe ferner ein zweiwertiges Kation umfaßt.

6. Verfahren nach Anspruch 1, worin der Verlängerungsbereich der Matrize eine Länge von mindestens etwa 10 Nukleotiden aufweist.

7. Verfahren nach Anspruch 6, worin der Verlängerungsbereich der Matrize im wesentlichen wiederholte Nukleotide umfaßt.

8. Verfahren nach Anspruch 1, worin die markierten Mononukleotide radioaktiv markiert sind.

9. Verfahren nach Anspruch 8, worin die Menge des markierten Polymeraseprodukts gemessen wird, indem die Probe, welche markiertes Polymeraseprodukt und überschüssiges markiertes Mononukleotid umfaßt, durch einen Filter geleitet wird, so daß das markierte Polymeraseprodukt auf dem Filter abgefangen wird, und die Menge der auf dem Filter befindlichen radioaktiven Markierung gemessen wird.

10. Verfahren nach Anspruch 9, worin der Filter ein Nylonfilter ist.

11. Verfahren nach Anspruch 1, worin die zu untersuchende Probe einen mutmaßlichen Inhibitor des Enzyms enthält.

12. Verfahren nach Anspruch 11, welches ferner den Vergleich der Menge an Enzymaktivität der Probe mit der Aktivität, die für eine Kontrollprobe, umfassend dasselbe Enzym, das nicht in Gegenwart eines mutmaßlichen Inhibitors vorliegt, bestimmt wurde, umfaßt.

13. Verfahren nach Anspruch 5, worin die zu untersuchende Probe einen mutmaßlichen Inhibitor von Ribozym-Aktivität enthält.

14. Verfahren nach Anspruch 13, welches ferner den Vergleich der Menge an Ribozym-Aktivität der Probe mit der Aktivität, die für eine Kontrollprobe, umfassend dasselbe Ribozym, das nicht in Gegenwart eines mutmaßlichen Inhibitors vorliegt, bestimmt wurde, umfaßt.

15. Verfahren nach Anspruch 1, worin das Enzym eine RNA-Endonuklease ist und die Probe ferner einen mutmaßlichen Inhibitor der Endonuklease umfaßt,
das Oligonukleotid-Substrat ein RNA-Molekül ist,
der 5'-Verlängerungsbereich mindestens 10 im wesentlichen wiederholte Nukleotide umfaßt,
das markierte Nukleotid radioaktiv markiert und komplementär zu den mindestens 10 im wesentlichen wiederholten Nukleotiden ist,
das markierte Polymeraseprodukt ein radioaktiv markiertes Polymeraseprodukt ist, welches die radioktive Markierung umfaßt,
und worin der Schritt (e) umfaßt
(i) Filtrieren der Probe, die das radioaktiv markierte Polymeraseprodukt und überschüssiges radioaktiv markiertes Mononukleotid umfaßt, durch Leiten der Probe durch einen Nylonfilter, so daß das radioaktiv markierte Polymeraseprodukt von dem Filter abgefangen wird; und
(ii) Messen der Menge an radioaktiv markiertem Polymeraseprodukt, die auf dem Filter abgefangen wurde, und Vergleichen dieser Menge mit derjenigen Menge, welche erhalten wird, wenn kein Endonuklease-Inhibitor in einer Kontrollprobe vorhanden ist.

16. Verfahren nach Anspruch 1, worin das Enzym ein Ribozym ist und die Probe ferner einen mutmaßlichen Inhibitor des Ribozyms umfaßt,
das Oligonukleotid-Substrat ein RNA-Molekül ist,
der 5'-Verlängerungsbereich mindestens 10 im wesentlichen wiederholte Nukleotide umfaßt,
das markierte Nukleotid radioaktiv markiert und komplementär zu den mindestens 10 im wesentlichen wiederholten Nukleotiden ist,
das markierte Polymeraseprodukt ein radioaktiv markiertes Polymeraseprodukt ist, welches die radioktive Markierung umfaßt,
die Probe zweiwertige Kationen für die Ribozym-Aktivität umfaßt und worin der Schritt (e) umfaßt
(i) Filtrieren der Probe, die das radioaktiv markierte Polymeraseprodukt und überschüssiges radioaktiv markiertes Mononukleotid umfaßt, durch Leiten der Probe durch einen Nylonfilter, so daß das radioaktiv markierte Polymeraseprodukt von dem Filter abgefangen wird; und
(ii) Messen der Menge an radioaktiv markiertem Polymeraseprodukt, die auf dem Filter abgefangen wurde, und Vergleichen dieser Menge mit derjenigen Menge, welche erhalten wird, wenn kein Ribozym-Inhibitor in einer Kontrollprobe vorhanden ist.

17. Verfahren zur Bestimmung der Stelle, an der eine Nuklease ein Oligonukleotid-Substrat spaltet, um ein einzelsträngiges Spaltprodukt zu erzeugen, umfassend:
a) Zurverfügungstellen von mindestens einer DNA-Matrize für eine Probe, wobei die DNA- Matrize einen ersten 3'-Abschnitt und einen 5'-Verlängerungsbereich umfaßt, unter der Voraussetzung, daß der 3'-Abschnitt für komplementär zu dem und von derselben Länge wie das Spaltprodukt erachtet wird und der 5'-Verlängerungsbereich mindestens ein Nukleotid umfaßt;
b) Zugeben von Spaltprodukt zu der Probe unter Hybridisierungsbedingungen zur Bildung einer RNA:DNA-Heteroduplex- oder DNA-DNA-Duplex-Struktur;
c) Zugeben eines markierten Mononukleotids, welches komplementär zu dem 5'-Verlängerungsbereich ist, zu der Probe;
d) Zugeben einer DNA-Polymerase unter Bedingungen, die das Stattfinden einer Polymerasereaktion erlauben, zu der Probe, wobei das markierte Mononukleotid dem 3'-Ende des Spaltprodukts hinzugefügt wird, falls der 5'-Abschnitt der DNA-Matrize komplementär zu dem und von derselben Länge wie das Spaltprodukt ist; und
e) Feststellen, ob die Reaktion von Schritt (d) stattfindet.

18. Verfahren nach Anspruch 17, worin eine Mehrzahl von DNA-Matrizen der Probe in Schritt (a) zur Verfügung gestellt wird, wobei die Mehrzahl von DNA-Matrizen jeweils den 5'-Verlängerungsbereich und einen zweiten 3'-Abschnitt umfasst, wobei sich der zweite 3'-Abschnitt in der Länge von dem ersten 3'-Abschnitt unterscheidet.

## Revendications

1. Procédé de mesure de l'activité d'une enzyme dans un échantillon, dans lequel ladite enzyme agit sur un substrat oligonucléotidique pour générer un produit oligonucléotidique simple brin comprenant :
a) l'ajout du substrat oligonucléotidique à l'échantillon à analyser pour générer le produit oligonucléotidique ;
b) l'hybridation du produit oligonucléotidique avec une matrice d'ADN, ladite matrice d'ADN comprenant un premier segment substantiellement complémentaire du produit oligonucléotidique et une région d'extension en 5' de la matrice attachée au premier segment, ladite région d'extension en 5' comprenant au moins un nucléotide, dans des conditions d'hybridation pour former une structure hétéroduplex ARN:ADN ou une structure duplex ADN:ADN ;
c) l'ajout d'un mononucléotide marqué qui est complémentaire de la région d'extension en 5' de la matrice d'ADN ;
d) l'ajout d'une ADN polymérase à la structure hétéroduplex ou duplex dans des conditions permettant à l'ADN polymérase de catalyser l'ajout du mononucléotide marqué à l'extrémité 3' du produit oligonucléotidique pour produire un produit de polymérase marqué ; et
e) la mesure de la quantité de produit de polymérase marqué en tant que mesure de la quantité d'activité de l'enzyme dans l'échantillon.

2. Procédé selon la revendication 1 dans lequel l'enzyme est une endonucléase.

3. Procédé selon la revendication 2 dans lequel le substrat est un substrat ADN.

4. Procédé selon la revendication 2 dans lequel le substrat est un substrat ARN.

5. Procédé selon la revendication 1 dans lequel l'enzyme est un ribozyme et l'échantillon contient en outre un cation divalent.

6. Procédé selon la revendication 1 dans lequel la région d'extension de la matrice a une longueur d'au moins environ 10 nucléotides.

7. Procédé selon la revendication 6 dans lequel la région d'extension de la matrice comprend des nucléotides substantiellement répétés.

8. Procédé selon la revendication 1 dans lequel les mononucléotides marqués sont radiomarqués.

9. Procédé selon la revendication 8 dans lequel la quantité de produit de polymérase marqué est mesurée en faisant passer l'échantillon contenant le produit de polymérase marqué et un excès de mononucléotide marqué à travers un filtre, de façon que le produit de polymérase marqué soit capturé dans le filtre, et en mesurant la quantité de radiomarqueur présent sur le filtre.

10. Procédé selon la revendication 9 dans lequel le filtre est un filtre de nylon.

11. Procédé selon la revendication 1 dans lequel l'échantillon à analyser contient un inhibiteur putatif de l'enzyme.

12. Procédé selon la revendication 11, comprenant en outre la comparaison de la quantité d'activité de l'enzyme dans l'échantillon à l'activité déterminée pour un échantillon témoin contenant la même enzyme qui n'est pas en présence d'un inhibiteur putatif.

13. Procédé selon la revendication 5 dans lequel l'échantillon à analyser contient un inhibiteur putatif d'activité de ribozyme.

14. Procédé selon la revendication 13, comprenant en outre la comparaison de la quantité d'activité de ribozyme dans l'échantillon à l'activité déterminée pour un échantillon témoin contenant le même ribozyme qui n'est pas en présence d'un inhibiteur putatif.

15. Procédé de la revendication 1, dans lequel ladite enzyme est une ARN endonucléase et ledit échantillon contient en outre un inhibiteur putatif de l'endonucléase,
ledit substrat oligonucléotidique est une molécule d'ARN,
ladite région d'extension en 5' comprend au moins 10 nucléotides substantiellement répétés,
ledit nucléotide marqué est radiomarqué et complémentaire desdits au moins 10 nucléotides substantiellement répétés,
ledit produit de polymérase marqué est un produit de polymérase radiomarqué comprenant ledit radiomarqueur,
et dans lequel ladite étape (e) comprend
(i) la filtration de l'échantillon contenant le produit de polymérase radiomarqué et un excès de mononucléotide radiomarqué par passage de l'échantillon à travers un filtre de nylon de façon que le produit de polymérase radiomarqué soit piégé par le filtre ; et
(ii) la mesure de la quantité de produit de polymérase radiomarqué piégée sur le filtre et la comparaison de cette quantité-là à celle obtenue lorsqu'aucun inhibiteur d'endonucléase n'est présent dans un échantillon témoin.

16. Procédé de la revendication 1, dans lequel ladite enzyme est un ribozyme et ledit échantillon contient en outre un inhibiteur putatif dudit ribozyme,
ledit substrat oligonucléotidique est une molécule d'ARN,
ladite région d'extension en 5' comprend au moins 10 nucléotides substantiellement répétés,
ledit nucléotide marqué est radiomarqué et complémentaire desdits au moins 10 nucléotides substantiellement répétés,
ledit produit de polymérase marqué est un produit de polymérase radiomarqué comprenant ledit radiomarqueur,
ledit échantillon contient des cations divalents pour l'activité du ribozyme, et dans lequel ladite étape (e) comprend
(i) la filtration de l'échantillon contenant le produit de polymérase radiomarqué et un excès de mononucléotide radiomarqué par passage de l'échantillon à travers un filtre de nylon de façon que le produit de polymérase radiomarqué soit piégé par le filtre ; et
(ii) la mesure de la quantité de produit de polymérase radiomarqué piégée sur le filtre et la comparaison de cette quantité-là à celle obtenue lorsqu'aucun inhibiteur de ribozyme n'est présent dans un échantillon témoin.

17. Procédé de détermination du site où une nucléase coupe un substrat oligonucléotidique pour créer un produit de coupure simple brin comprenant :
a) l'introduction d'au moins une matrice d'ADN dans un échantillon, où la matrice d'ADN comprend un premier segment en 3' et une région d'extension en 5', pour autant que le segment en 3' est censé être complémentaire du produit de coupure et de même longueur que ce dernier, et ladite région d'extension en 5' comprend au moins un nucléotide ;
b) l'ajout du produit de coupure à l'échantillon dans des conditions d'hybridation pour former une structure hétéroduplex ARN:ADN ou une structure duplex ADN:ADN ;
c) l'ajout à l'échantillon d'un mononucléotide marqué qui est complémentaire de la région d'extension en 5' ;
d) l'ajout à l'échantillon d'une ADN polymérase dans des conditions permettant à une réaction de polymérase de se produire dans laquelle le mononucléotide marqué est ajouté à l'extrémité 3' du produit de coupure si le segment en 5' de la matrice d'ADN est complémentaire du produit de coupure et de même longueur que ce dernier ; et
e) la détermination de la survenue ou non de la réaction de l'étape d).

18. Procédé selon la revendication 17 dans lequel une pluralité de matrices d'ADN sont introduites dans l'échantillon dans l'étape (a), où la pluralité de matrices d'ADN comprennent chacune ladite région d'extension en 5' et un second segment en 3', où ledit second segment en 3' diffère en longueur dudit premier segment en 3'.


### SEQUENCE LISTING

(1) GENERAL INFORMATION:
   (i) APPLICANT: MERCK & CO., INC.
      Cole, James L.
      Olsen, David B.
      Kuo, Lawrence C.
   (ii) TITLE OF INVENTION: DNA POLYMERASE EXTENSION ASSAY
   (iii) NUMBER OF SEQUENCES: 5
   (iv) CORRESPONDENCE ADDRESS:
      (A) ADDRESSEE: Ms. Joanne J. Giesser
      (B) STREET: 126 E. Lincoln Avenue, P.O. Box 2000-0907
      (C) CITY: Rahway
      (D) STATE: New Jersey
      (E) COUNTRY: USA
      (F) ZIP: 07065
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Giesser, Joanne M.
      (B) REGISTRATION NUMBER: 32,838
      (C) REFERENCE/DOCKET NUMBER: 19398 PCT
   (ix) TELECOMMUNICATION INFORMATION:
      (A) TELEPHONE: (908)-594-3046
      (B) TELEFAX: (906)-594-4720
(2) INFORMATION FOR SEQ ID NO:1:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (iii) HYPOTHETICAL: NO
   (iv) ANTI-SENSE: NO
   (xi) SEQUENCE DESCRIPTION: SEQ ID NO:1:
   GUUUUUAUUU UUAAUUUUC 19
(2) INFORMATION FOR SEQ ID NO:2:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (iii) HYPOTHETICAL: NO
   (iv) ANTI-SENSE: NO
   (xi) SEQUENCE DESCRIPTION: SEQ ID NO:2:
   ACACUUGCUU UUG 13
(2) INFORMATION FOR SEQ ID NO:3:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (iii) HYPOTHETICAL: NO
   (iv) ANTI-SENSE: NO
   (xi) SEQUENCE DESCRIPTION: SEQ ID NO:3:
   GTTTTTATTT TTA 13
(2) INFORMATION FOR SEQ ID NO:4:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (iii) HYPOTHETICAL: NO
   (iv) ANTI-SENSE: NO
   (xi) SEQUENCE DESCRIPTION: SEQ ID NO:4:
   CCCCCCCCCC TAAAAATAAA AAC 23
(2) INFORMATION FOR SEQ ID NO:5:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (iii) HYPOTHETICAL: NO
   (iv) ANTI-SENSE: NO
   (xi) SEQUENCE DESCRIPTION: SEQ ID NO:5:
   CCCCCCCCCC TTAAAAATAA AAAC 24